# EUROPEAN PATENT APPLICATION

(11) **EP 1 591 126 A2**
(43) Date of publication of application: **02.11.2005**
(21) Application number: 05002297.9
(22) Date of filing: 03.02.2005
(51) Int. Cl.: A61K 35/78, A61P 35/00, A61P 37/04, A61P 25/00, A61P 13/00

(54) **Pharmaceutical composition containing powder or extract of a parasite plant of the Loranthaceae family**

(30) Priority: 04.02.2004 JP 2004027440
(71) Applicant: Kawamura, Etsuko Pamudji, Jakarta (ID)
(72) Inventor: Kawamura, Etsuko Pamudji, Jakarta (ID)
(74) Representative: Forstmeyer, Dietmar

(57) **Abstract**

This invention is to provide a health food and a pharmaceutical preparation especially useful for cancer treatment, as pain reliever, as diuretic and for enhancing immunity. A health food and a pharmaceutical preparation containing at least one of the plants of Scrrula genus of Loranthaceae family; the plants of Dendrophthoe genus of Loranthaceae family; the plants of Macrosolen genus of Loranthaceae family; the plants of Lepeostegeres genus of Loranthaceae family or the plants of Barathranthus genus of Loranthaceae family, which are native in the tropical region, or dried one thereof; or a extract thereof.

## Description

This invention is related to a health food and a pharmaceutical preparation containing a native plant of Loranthaceae family of the tropical region (hereinafter stated as "Tropical Mistletoe") or an extract thereof.

The nature-loving movement together with the health boom in the recent years pushes ahead more kinds of health foods and pharmaceutical preparations made from herbal plants introduced to the world.

For example, JP2535311 declares an invention of a health tea, the main ingredient of which is sword bean(Canavalia gladiata, (Japanese name: Natamame)) which has an effect on hiccup, swollen or hurting throat, hoarse voice, mouth inflammation, constipation, diarrhea, stomach pain, hyposthenia after illness, anal fistula, empyema, alveolar pyorrhea, furuncle, tonsillitis, eczema and other skin diseases as well as strengthening kidney function.

JP2856372 declares an invention of a health drink containing an extract of Sasa veitchii (Japanese name: Kumazasa) which has an effect on gastrointestinal disease, cold, diabetes, high pressure, inflammatory disease, decline of fever for cold, tonsillitis, mouth inflammation, asthma and others; as well as an extract of aloe which is antibacterial, antifungal, antitumor and antiulcer.

JP3180264 declares an invention of a health drink of which raw material is greater galangal (Languas galanga, Japanese name: Nankyo) and lemongrass (Cymbopogan citrtus), which have a cancer suppressing effect.

Further JP3159508 declares an invention of a health food and a pharmaceutical preparation consisting of an extract of oolong stem tea, which has an effect on inflammation at pharynges and allergic reaction.

Further JP H07-2685A, JP 2001-502305A and JP H11-503453A declare use of a Viscum album extract for pharmaceutical preparation.

JP H07-2685A states that a lectin contained in viscum album extract increases immunity, JP 2001-502305A states that a Viscum extract is parenterally used as an immunity activator, and JP H11-503453A states that an extract of Viscus album is useful for immunity suppressing diseases such as AIDS, cancer.

More over, an extract of Viscus album is introduced in the market in Germany as a product called Iscador™.

However, all these extracts are of Viscus album, a native plant of European region and a parenteral medicine where the active ingredient is a lectin.

### Problems to be solved

A health food or a pharmaceutical preparation of which material is a native plant of Loranthaceae family of the tropical region, such as Scrrula genus of Loranthaceae family; Dendrophthoe genus of Loranthaceae family; Macrosolen genus of Loranthaceae family; Lepeostegeres genus of Loranthaceae family or Barathranthus genus of Loranthaceae family, has never been provided before.

As a specimen of Scrrula genus of Loranthaceae family, Scurrula longicerifolius (Hayata) Danser (Japanese name: Nindoubano Yadorigi) is known to inhabit in Okinawa, Japan. However this specimen is so scarce that it faces a danger of extinction. There has never been a health food or a pharmaceutical preparation made from this specimen.

### Means to solve the problems

This invention is to provide a health food and a pharmaceutical preparation of which material is taken from a native plant of Loranthaceae family of the tropical region.

The active agents of Tropical Mistletoes are flavonoid, fatty acid and hexadeca-8,10,12-trienoic acid which are different from that of Viscus album (hereinafter stated as European Mistletoe) which is lectin.

### Advantageous effect of the Invention

The decoction of this invention has a light taste like a coarse tea and is pleasant to taste. When the same decoction is given to patients of tumor, hepatitis, and cancer, symptomatic relieves are recognized. To explain more specifically, a patient of tumor, who took 250 ml of a decoction of the working example as stated below three times a day after meal, gradually found relief within 40 to 60 days and after continuous drink of the decoction finally the tumor disappeared. This decoction can be taken by any healthy body and is expected to contribute to enhance immunity and to maintain healthy conditions.

Thus, a health food of this invention not only provides a light and pleasant taste like a coarse tea but also is expected to perform a remarkable effect to strengthen immunity. A pharmaceutical preparation of this invention is expected to perform an effect to strengthen immunity and to provide symptomatic relief from tumor and such. It also has an effect on pain relieving and diuresis. More over a pharmaceutical preparation of this invention has very slight side effect and high safety.

### Best embodiment of the implementation of the invention

The plant of Loranthaceae family used for this invention can be chosen from Scrrula genus, Dendrophthoe genus, Macrosolen genus, Lepeostegeres genus or Barathranthus genus. Preferably, Scurrula antropurpurea or Scurrula lepidota as a specimen of Scrrula genus, Dendrophthoe pentandra as a specimen of Dendrophthoe genus, Macrosolen cochincinensis as a specimen of Macrosolen genus, Lepeostegeres gemmiflopud as a specimen of Lepeostegeres genus, or Barathranthus axanthus as a specimen of Barathranthus genus is used. All of these specimens inhabit and are available in Jawa Island, Indonesia.

This invention can be produced by the following method. First, the plant of the raw material is divided into leaves, stems and flowers. Leaves are washed twice. Stems are also washed twice and cut into an appropriate size for handling. Flowers are not necessary washed.

25 grams of dried leaves, stems and flowers are put into 2 liters of water and brewed until water volume reduced to 1500 ml. The decoction obtained in such a manner is already ready to drink.

Another embodiment of implementation of the invention is that the plant of the raw material is not dried before put into the water to be brewed.

Still another embodiment of implementation of the invention is that the dried plant of the raw material is divided into sachets before put into the water to be brewed.

### Working Examples

Concrete working examples are explained below. In these examples, leaves, stems or flowers of Scrrula atropurpurea of Scrrula genus, Loranthaceae family; Scrrula lepitoda of Scrrula genus, Loranthaceae family; Dendrophthoe pentandra of Dendrophthoe genus, Loranthaceae family; Macrosolen cochinchinensis of Macrosolen genus, Loranthaceae family; Lepeostegeres gemmiflopus of Lepeostegeres genus, Loranthaceae family; or Barathranthus axanthus of Barathranthus genus, Loranthaceae family are used as raw materials.

The 1st working example of the invention is obtained by brewing 25 grams of dried leaves, stems or flowers of Macrosolen cochinchinensis in 2 liters of water until the water volume reduced to 1500 ml.

The 2nd working example of this invention is obtained by brewing the compound material of the components shown in Table 1 in 2 liters of water until the water volume reduced to 1500 ml.

**Table 1**

| | | |
|---|---|---|
| 1 | Dried leaves, stems or flowers of Scrrula atropurpurea | 5.2 g |
| 2 | Dried leaves, stems or flowers of Scrrula lepitoda | 8.4 g |
| 3 | Dried leaves, stems or flowers of Dendrophthoe pentandra | 8.2 g |
| 4 | Dried leaves, stems or flowers of Macrosolen cochinchinensis | 1.5 g |
| 5 | Dried leaves, stems or flowers of Lepeostegeres gemmiflopus | 1.4 g |
| 6 | Dried leaves, stems or flowers of Barathranthus axanthus | 0.3 g |

Further, the 3rd working example is a tea bag containing 35 +-2g of the compound materials of the components shown in Table 2.

**Table 2**

| | | |
|---|---|---|
| 1 | Dried leaves, stems or flowers of Scrrula atropurpurea | 10.0 g |
| 2 | Dried leaves, stems or flowers of Scrrula lepitoda | 10.0 g |
| 3 | Dried leaves, stems or flowers of Dendrophthoe pentandra | 12.0 g |
| 4 | Dried leaves, stems or flowers of Macrosolen cochinchinensis | 1.4 g |
| 5 | Dried leaves, stems or flowers of Lepeostegeres gemmiflopus | 1.2 g |
| 6 | Dried leaves, stems or flowers of Barathranthus axanthus | 0.4 g |

These working examples are not to eliminate the coverage of the invention. The invention can be implemented by using a different component from the above-mentioned components, a different combination of different specimens from the above-mentioned recipes, or a different method from the above-mentioned methods.

The following is the organic solvent extraction result of the working example 3 of the invention.

**Table 3**

| Active substances | Contents (g) | (%) |
|---|---|---|
| Total flavonoid | 42.2 | 41.6 |
| Tannin | 7.3 | 6.7 |
| Polysaccharide | 30.1 | 29.5 |
| Methylripariochomene A | 4.6 | 4.2 |
| Hexadeca-8,10,12-trienoic acid | 15.8 | 17.9 |
| Total | 100.0 | 100.0 |

100 g of this compound contains 41.6 % of flavonoid, 29.5% Polysaccharide and 17.9% of a particular fatty acid namely Hexadeca-8, 10, 12-trienoic acid.

On the other hand, lectin, viscotoxin and alkaloid are detected from Iscador ™.

An advantageous effect of the invention to increase immunity is explained by showing the result of tests as below.

### Test on Reproduction of ROI (Rective Oxygen Intermediate)

### <Testing Method>

An ROI reproduction test is performed by using 70 pieces of tumor mice. These mice are Swiss outbred adults with body weight 20 to 25 grams, and are made to have tumor by inducing benzo (a) pyrene dissolved by Oleum Olivarium solution.

The tested mice are divided into 7 groups as shown in Table 4. The 1st group is given distilled water as control. The 2nd group is given 0.5 ml of 10% decoction of Macrosolen cochinchinensis, the 3rd group 1ml of 20% decoction of Macrosolen cochinchinensis, the 4th group 1ml of 30% decoction of Macrosolen cochinchinensis, the 5th group 0.5ml of 10% decoction of the compound material of Table 1, the 6th group 1ml of 20% decoction of the compound material of Table 1, the 7th group 1ml of 30% decoction of the compound material of Table 1 each day for 15 days.

Here, 10% decoction means a decoction obtained by brewing 50 grams of dried material in 500 ml of distilled water under 90 °C stirred for 15 minutes then filtering the decoction while it is warm and adding some water through the filter until the volume of the decoction reaches 500ml. 20 % decoction is obtained by cooking 100 ml of the 10 % decoction until condensed to 50 ml. 30 % decoction is obtained by cooking 150 ml of the 10 % decoction until condensed to 50 ml.

**Table 4**

| | Treatments | Doses (ml) | Periods (days) | |
|---|---|---|---|---|
| Group 1 | Distilled water | | 15 | control |
| Group 2 | Macrosolen cochincinensis 10% | 0.5 | 15 | |
| Group 3 | Macrosolen cochincinensis 20% | 1 | 15 | |
| Group 4 | Macrosolen cochincinensis 30% | 1 | 15 | |
| Group 5 | Compound of Table 1 10% | 0.5 | 15 | |
| Group 6 | Compound of Table 1 20% | 1 | 15 | |
| Group 7 | Compound of Table 1 30% | 1 | 15 | |

### <Judging Method>

15 days later, the mice are killed by narcoses using chloroform. The abdomen skin is opened so that the peritoneum cover is visible. About 8 ml medium of RPMI-1640 is injected to the peritoneum cavity, wait for 3 minutes while giving pressure by fingers, then the medium is aspirated. The obtained cells are centrifuged at 1200 rpm, 4 degrees Celsius for 10 minutes. After the supernatant is discarded, the pellet is resuspended into a complete medium. Then cells are counted by hemocytometer. Cell viability is determined by trypan blue, then resuspended by the complete medium so it is reached density of 2.5 x 10⁵ per ml. Further cultivated with CO₂ 25% at temperature 37 °C for 24 hours. ROI content is counted based on reduction power of macrophage toward Nitroblue Tetrasolium (NBT reduction assay) . By the existence of superoxide anion, NET is oxidized to form undissolved formazan precipitate. To induce secretion of superoxide anion, cell culture is stimulated by Phorbol 12-Myristate 13-acetate (PMA) with final concentration 125 ng/ml.

Macrophage culture is washed twice with RPMI 1640 then added with 500 µl of Nitroblue solution containing 125 ng/ml of PMA then culture is incubated at temperature 37°C with 5% of CO₂ for 60 minutes. The incubation result is washed three times with PBS then dried at room temperature and fixated with absolute methanol for 30 seconds. After the methanol is dried, the preparation is smeared with 2% of neural red for 15 seconds then dried at room temperature. Percentage of the cells showing NBT reduction in 200 cells is counted on light microscope with 400x enlargement.

### <Judging Result>

As a result of the test, the percentages of cells indicating NBT reaction in every 200 macrophages are shown in Table 5.

**Table 5**

| | Treatments | Doses (ml) | Periods (days) | ROI (%) |
|---|---|---|---|---|
| Group 1 | Distilled water | | 15 | 15.7 |
| Group 2 | Macrosolen cochincinensis 10% | 0.5 | 15 | 18.3 |
| Group 3 | Macrosolen cochincinensis 20% | 1 | 15 | 46.0 |
| Group 4 | Macrosolen cochincinensis 30% | 1 | 15 | 35.6 |
| Group 5 | Compound of Table 1 10% | 0.5 | 15 | 22.1 |
| Group 6 | Compound of Table 1 20% | 1 | 15 | 44.7 |
| Group 7 | Compound of Table 1 30% | 1 | 15 | 42.3 |

As we can see at Table 5, ROI of mice given either Macrosolen cochincinensis or the compound of Table 1 increased. This phenomenon is more obvious when more than 20 % of decoctions are given to mice.

### Test on Helper T lymphocyte activation

### <Judging Method>

From the same mice that the macrophages are taken from the peritoneum, further lymph tissue thereof is taken. The lymph tissue is ripped then filtrated using nylon filter. Erythrocyte is lysed by TRIS ammonium chloride for 10 minutes. The cells of filtration result is washed twice using HBBS and put into L-arginine without RPMI with 10% of Fetal Calf Serum (FCS), 1 mol/L of glucose and 40 µmol/L of 2-mercaptoethanol. Cell viability is counted by trypan blue in Haemocytometer of Neubauer. Furthermore, proliferation activity of lymphocyte culture is stimulated by anti-CD3 monoclonal (SIGMA Catalog No. F 7275) and incubated for 24 hours. Furthermore culture is stored at frozen temperature to be performed counting of IL-2. Activation of helper T lymphocyte is judged based on the counted number of IL-2.

Content of Interleukin-2 in cell supernatant is counted using Elisa method as follows. Microplate with 96 holes is sensitized with sample which has been diluted using PBS and 10% of Fetal Calf Serum (FCS) for one night. Then it is added anti mouse IL-2 (mouse IgG1, clone 5344-111; Becton Dickinson) which is diluted with 50 µl of Tris HCl pH 9.5. After incubation for about one hour, it is washed with PBS and 10% of Fetal Calf Serum (FCS) three times. Afterwards, plate is incubated using secondary antibody labeled biotynil and HVR Avidin. Then absorbance is measured by using ELISA reader at 406 nm.

### <Judging Result>

The densities of IL-2 of mice lymph of each group obtained from the absorption measurement mentioned above are shown in Table 6.

**Table 6**

| Groups | Treatments | Dose (ml) | Period (days) | IL-2 density (ng/l) |
|---|---|---|---|---|
| Group 1 | Distilled water | | 15 | 1.49 |
| Group 2 | Macrosolen cochincinensis 10% | 0.5 | 15 | 1.41 |
| Group 3 | Macrosolen cochincinensis 20% | 1 | 15 | 3.21 |
| Group 4 | Macrosolen cochincinensis 30% | 1 | 15 | 2.72 |
| Group 5 | Compound of Table 1 10% | 0.5 | 15 | 1.29 |
| Group 6 | Compound of Table 1 20% | 1 | 15 | 3.03 |
| Group 7 | Compound of Table 1 30% | 1 | 15 | 3.74 |

As we can see at Table 6, the mice given more than 20 % decoction of either Macrosolen cochincinensis or the Compound of Table 1 significantly increased IL-2 density and thus activation of Helper T lymphocyte thereof is observed.

### Cancer treatment effect

The reaction of cancer patients who used the tea bag of the 3rd working example is explained below.

### (1) Brewing

1) Put the teabag of Table 3 into 2 liters of boiled water and brew by a small fire for 20 to 25 minutes until the volume of water reduces to 1.5 liters.
2) Remove the teabag out and cool down.

### (2) Doses

Patients take 250 ml of the decoction 3 times a day after meal until the cancer disappears, shrikes or decreases. In order to avoid recurrence, 250 ml after evening meal is sufficient.

### (3) Result

31 patients shown in Table 7 took the decoction.

**Table 7**

| No | Sex | Age | Cancer area | Stage | No | Sex | Age | Cancer area | Stage |
|---|---|---|---|---|---|---|---|---|---|
| 1 | F | 46 | Breast | IV | 17 | F | 38 | Left breast cancer | III |
| 2 | F | 46 | Left breast | IIa | 18 | F | 18 | Ovarian | III |
| 3 | M | 68 | Pancreas | IV | 19 | F | 43 | Uterine cancer | IV |
| 4 | F | 52 | Right breast | II | 20 | F | 37 | Uterine | IV |
| 5 | F | 29 | Pharynx | IIIT 1b | 21 | M | 59 | Prostatic | IIIa |
| 6 | F | 45 | Right breast | II | 22 | M | 67 | Lung | IV |
| 7 | F | 43 | Right breast | II | 23 | F | 43 | Uterine | III |
| 8 | M | 48 | Prostatic | Iia | 24 | M | 52 | Stomach | IIIb |
| 9 | F | 38 | Lung | IV | 25 | F | 72 | Colon | IV |
| 10 | F | 49 | Uterine | IIIa | 26 | F | 39 | Right breast | III |
| 11 | M | 60 | Stomach | IIIb | 27 | F | 64 | Lung | IV |
| 12 | F | 56 | Uterine | IIIb | 28 | F | 36 | Uterine | IV |
| 13 | F | 41 | Neck | II | 29 | M | 47 | Rectum | III |
| 14 | F | 30 | Lung cancer | IV | 30 | F | 53 | Lung | IV |
| 15 | F | 50 | Stomach | IIIa | 31 | F | 55 | Right breast r | III |
| 16 | F | 55 | Left breast cancer | IV | | | | | |

The results are shown in Table 8. Here, the criteria for the judgment are according to the Japanese Cancer Association.

**Table 8**

| Judgment | Number of cases | Reaction (%) | Case Numbers |
|---|---|---|---|
| CR | 5 | 16.1 | 9,18,21,22,30 |
| PR | 7 | 22.6 | 8,10,11,19,26,28,31 |
| NC | 2 | 6.4 | 3,6 |
| PD | 0 | 0.0 | - |
| Judgment Impossible | 2 | 6.4 | 16,29 |
| Judgment Unclear (all cases alive) | 15 | 48.4 | 1,2,4,5,7,12,13,14,15, 17,20,23,24,25,27 |
| Total | 31 | 100.0 | 31 cases |
| CR: Complete Reaction (Tumor disappeared for 4 weeks or more) | | | |
| PR: Partial Reaction (More than 50% of Tumor disappeared for 4 weeks) | | | |
| NC: No Change (Shrink of Tumor 50% or more, Enlarge of Tumor 25% or less for 4 weeks) | | | |
| PD: Progressive Disease (Enlarge of Tumor 25 % or more) | | | |

As a result, the reaction of the decoction is CR 16.1%, PR 22.6%, totally 38.7%.

### Clinical Characteristics

### (1) Pain relieving effect

Among the cases shown in Table 7, 54.8% of cases (17 cases out of 31) reported pain before taking the decoction, among which 82.4% cases (14 cases out of 17) reported pain relieving effect after taking the decoction. This rate is high.

Further, pain relieving cases of each disease stage are 41.2% for Stage IV, 35.3% Stage III, 5.9% Stage II. Pain relieving effect of the decoction is more obvious in the expanded stage of disease.

As a result of the research on the pain relieving effect of the decoction by using an animal model, equivalent pain relieving effect to aspirin was confirmed. Therefore it is assumed that this decoction acts in suppression of acceptors which are the cause of pain. It is suspected total flavonoid may related to this phenomenon as active agents of the decoction.

### (2) Diuresis effect

Among the cases of Table 7, 58.1% (18 cases out of 31) reported increase of volume of urine a day. Among these 18 cases, the average volume of urine was 900 ml before taking the decoction and increased to 2,200 ml after 1 month from starting to take the decoction.

It is assumed that this diuresis effect is caused by tannin (catechin) or total flavonoid as active agents of the decoction.

### Side Effects

The side effects of the decoction reported from the 31 cases of Table 7 are only related to digestive organs as shown in Table 9.

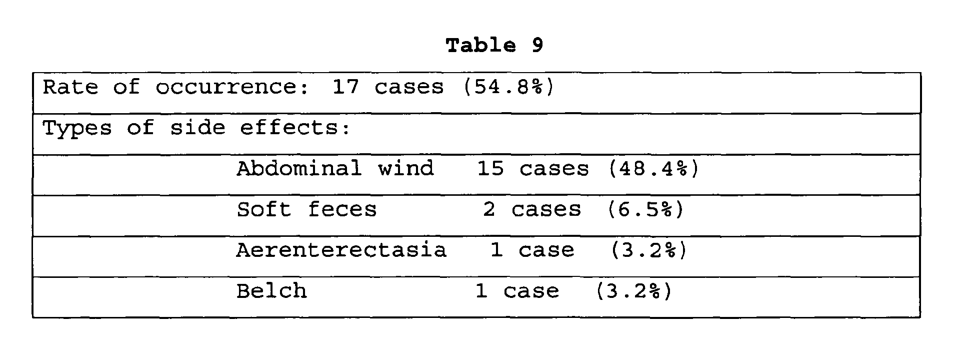

Abdominal winds of the highest rate 48.4% were assumed to be caused by the dietary instruction to take more grains, beans and vegetables (food fibers) than meat.

As contrasted to the product which is sold as Iscador™ in Germany is known to cause side effects such as anaphylaxy, headache, fever, chill, nausea, vomiting, stomach pain; no severe side effects are observed in the decoction of the invention.

Besides it, the decoction of the invention has no contraindication while Iscador™ contraindicate use in antidepressant drugs (MOA inhibitor), high pressure, pregnant, young children.

## Claims

1. A health food containing at least one of the plants of Scrrula genus of Loranthaceae family, the plants of Dendrophthoe genus of Loranthaceae family, the plants of Macrosolen genus of Loranthaceae family, the plants of Lepeostegeres genus of Loranthaceae family or the plants of Barathranthus genus of Loranthaceae family, which are native in the tropical region, or dried one thereof; or an extract thereof.

2. A health food according to Claim 1 which is used for enhancing immunity.

3. A pharmaceutical preparation containing at least one of the plants of Scrrula genus of Loranthaceae family, the plants of Dendrophthoe genus of Loranthaceae family, the plants of Macrosolen genus of Loranthaceae family, the plants of Lepeostegeres genus of Loranthaceae family or the plants of Barathranthus genus of Loranthaceae family, which are native in the tropical region, or dried one thereof; or an extract thereof.

4. A pharmaceutical preparation according to Claim 3 which is used for enhancing immunity.

5. A pharmaceutical preparation according to Claim 3 which is used for cancer treatment.

6. A pharmaceutical preparation according to Claim 3 which is used as pain reliever.

7. A pharmaceutical preparation according to Claim 3 which is used as diuretic.

8. A pharmaceutical preparation according to one from Claims 3 to 7, which is for oral intake.
